# EUROPEAN PATENT SPECIFICATION

(11) **EP 2 134 742 B1**
(45) Date of publication and mention of the grant of the patent: **27.06.2012**
(21) Application number: 08730629.6
(22) Date of filing: 25.02.2008
(51) Int. Cl.: C07K 14/20

(54) **IMMUNOGENIC COMPOSITIONS FOR TREATING AND PREVENTING ANIMAL INFECTIONS**
IMMUNOGENE ZUSAMMENSETZUNGEN ZUR BEHANDLUNG UND PRÄVENTION VON TIERINFEKTIONEN
COMPOSITIONS IMMUNOGÈNES DE TRAITEMENT ET DE PRÉVENTION D'INFECTIONS CHEZ LES ANIMAUX

(30) Priority: 27.02.2007 US 903586 P
(43) Date of publication of application: 23.12.2009
(73) Proprietor: Wyeth LLC, Madison, NJ 07940 (US)
(72) Inventor: GILL, Michael, A., Sioux Falls SD 57108-5063 (US)
(74) Representative: Murnaghan, Stephen
(86) International application number: PCT/US2008/054867
(87) International publication number: WO 2008/106393

(56) References cited:
- WO-A-2006/038115
- GEORGE E. MOORE: "Canine leprosis , United States, 2002-2004" INTERNET ARTICLE, [Online] March 2006 (2006-03), XP002494913 Retrieved from the Internet: URL:http://findarticles.com/p/articles/mi_ m0GVK/is_3_12/ai_n16109014/print?tag=artBo dy;col1> [retrieved on 2008-09-04]
- ADIN C A ET AL: "Treatment and outcome of dogs with leptospirosis: 36 cases (1990-1998)." JOURNAL OF THE AMERICAN VETERINARY MEDICAL ASSOCIATION 1 FEB 2000, vol. 216, no. 3, 1 February 2000 (2000-02-01), pages 371-375, XP009105300 ISSN: 0003-1488
- ANONYMOUS: "Introduction of Leptovax 4 canine vaccine" INTERNET ARTICLE, [Online] May 2005 (2005-05), XP002494914 Retrieved from the Internet: URL:http://www.wyethah.ca/article.asp?page id=prlepto4_0505> [retrieved on 2008-09-04]

## Description

### TECHNICAL FIELD

The present disclosure relates to compositions and methods that provide treatment or protection against viral and bacterial infections in animals. More specifically, the present disclosure relates to multivalent vaccine compositions for treating or preventing diseases or disorders in canines caused by or associated with one or more of canine distemper virus, canine adenovirus (type 1 or type 2), canine parainfluenza virus, canine parvovirus, *Leptospira canicola, Leptospira icterohaemorrhagiae, Leptospira grippotyphosa, Leptospira pomona, Leptospira bratislava, Leptospira hardjo, Leptospira autumnalis, Leptospira australis, Leptospira hebdomadis,* and *Bordetella bronchiseptica.* The Leptosira compositions can also be used for treating or preventing diseases or disorders in canine, swine, and bovine.

### BACKGROUND

Leptospirosis is a contagious disease affecting both animals and humans. The infection is caused by a bacterial pathogen called Leptospira. Bacteria of the genus Leptospira are spirochaetes, which can be pathogenic and include several known species and a large number of serovars. Exemplary pathogenic species include *L. interrogans, L. inadai, L. borgpetersenii, L. santarosai, L. kirschneri, L weilii* or *L. noguchii.* Pathogenic Leptospira serovars occur naturally in a large variety of livestock animals, companion animals, wild animals, and humans. The host range of Leptospira serovars is generally quite broad, but different symptoms will result depending on the host infected. For example, leptospirosis in dogs may result in chronic liver and kidney disease, and in death.

The course and severity of the leptospiral disease is often dependent upon the serovar responsible for the infection. Serovars associated with liver infection and symptoms of urine discoloration and/or jaundice (icterus), elevated liver enzymes, and gastrointestinal symptoms, include *L*. *icterohaemorrhagiae* and *L. grippotyphosa.* The serovar *L*. *grippotyphosa* is also associated with symptoms of renal failure, as is the serovar *L. pomona.* As recently as the 1980s, *L. icterohaeniorrhagiae* and *L*. *canicola* were identified as the most prevalent serovars causing leptospirosis in canines. By the 1990s, however, an increased incidence oi *L. grippotyphosa* and *L. pomona* was observed in conjunction with a resurgence of leptospirosis disease suggesting a changing trend in the epidemiology of this disease. Some additional pathogenic leptospira serovars are beginning to emerge as medically relevant, such as *L*. *bratistava, L. autumnalis, L. australis,* and *L*. *hebdomadis.*

Leptospira serovars are classified based upon the types of antigens on their surface (*i.e*., cell-surface markers that elicit antibody produclion), but these cell surface antigens provide little cross-immunity between serovars. Thus, vaccinating against certain serovars does not afford protection against other serovars. Moreover. vaccines that combine antigens from several serovars, or antigens from other organisms (*e.g*., virus), may result in antigen interference - that is, a failure of one or more of the combined antigens to elicit an immune response because of a "masking" effect caused by the other antigens in the composition.

Therefore, there is a need for the development of effective compositions and methods to treat and prevent disease or disorders caused or associated with Leptospira infection.

WO 2006/038115 discloses multivalent canine vaccines against Leptospira bratislava and other pathogen.

Moore et al: "Emerging Infectious Diseases"; 2006 March; 12(3):501-3, relates to a study on "Canine leptospirosis, United States, 2002-2004".

Adin et al: "Journal of the American Veterinary Medical Association", vol. 216, no. 3,1 February 2000, pages 371-375, relates to the "Treatment and outcome of dogs with leptospirosis: 36 cases (1990-1998)."

An anonymously authored internet article: found at http://www.wyethah.ca/article.asp?page id=prlepto4_0505>, relates to "Introduction of Leptovax 4 canine vaccine".

### SUMMARY

The present invention provides immunogenic compositions comprising a Leptospira cell surface immunogen or an inactivated whole or partial cell preparation of *Leptospira bratislava. Leptospira autumnalis, Leptospira australis,* and *Leptospira hebdomadis.* In certain embodiments the compositions of the invention comprise one or more outer membrane capsular proteins (OMC) derived from *Leptospira bratislava, Leptospira autumnalis, Leptospira australis,* and/or *Leptospira hebdomadis.* The invention also provides immunogenic compositions comprising a Leptospira cell surface immunogen or an inactivated whole or partial cell preparation of *Leptospira bratislava, Leptospira autumna*/*is. Leptospira australis*, and *Leptospira hebdomadis,* and further comprising one or more viral immunogen selected from the group consisting of canine distemper (CD) virus, an attenuated strain of canine adenovirus type-2 (CAV-2), an attenuated strain of canine parainfluenza (CPI) virus, an attenuated strain of canine parvovirus (CPV), and an inactivated strain of canine coronavirus (CCV). The immunogenic compositions of the present invention, in certain embodiments, may also comprise a Leptospira cell surface immunogens or an inactivated whole or partial cell preparation derived from one or more Leptospira strains selected from the group consisting of *L*. canicola, *L. grippotyphosa. L icterohaemorrhagiae. L. pomona,* and *L*. *hardjo*. In certain embodiments, the immunogenic compositions of the invention may include at least one *Bordetella bronchiseptica* immunogens such as, *e.g.,* the p68 *Bordetella* surface protein.

These and other embodiments, features, and advantages of the disclosure will become apparent from the detailed description and the appended claims set forth herein below.

### DETAILED DESCRIPTION

The present disclosure provides a variety of multivalent immunogen compositions for treating or preventing bacterial infections or both bacterial and viral infections. This disclosure, therefore relates generally to the surprising discovery that a different combination of Leptospira cell surface immunogens could be combined to create an immunogenic composition that does not result in antigenic interference. That is, each individual immunogen of the composition is capable of eliciting an immune response, particularly a protective immune response, with this activity being unaffected by the presence of other antigens. More specifically, one embodiment of the immunogenic composition comprises a Leptospira cell surface immunogen of *Leptospira bratislava, Leptospira autumnalis, Leptospira australis*, and *Leptospira hebdomadis.* In addition, these Leptospira immunogens are just as immunogenic when combined with other viral antigens, including a canine distemper (CD) virus antigen, a canine adenovirus type 2 (CAV-2) antigen, a canine parainfluenza (CPI) virus antigen, a canine parvovirus (CPV) antigen, a canine coronavirus (CCV) antigen, and any combination thereof. Additionally, an antigen from *Bordetella bronchispetica,* such as p68 (a *Bordetella* surface protein), can also be added to the immunogenic composition. These immunogenic compositions can be used as a vaccine to treat or ameliorate or prevent various Leptospira infections and various viral infections. Discussed in more detail below are multivalent immunogen compositions suitable for use within the present disclosure, as well as representative therapeutic or veterinary uses.

Prior to setting forth the disclosure in more detail, it may be helpful to an understanding thereof to set forth definitions of certain terms to be used hereinafter.

As described herein, any concentration range, percentage range, ratio range or integer range is to be understood to include the value of any integer within the recited range and, when appropriate, fractions thereof (such as one tenth and one hundredth of an integer), unless otherwise indicated. As used herein, "about" or "comprising essentially of" mean ±15% of the indicated value or range, unless otherwise indicated. The use of the alternative (*e.g*., "or") should be understood to mean either one, both, or any combination thereof of the alternatives.

The term "immunogen" or "antigen," as used herein, refers to an agent that is recognized by the immune system when introduced into a subject and is capable of eliciting an immune response. In certain embodiments, the immune response generates is a protective immune response. Immunogens include "surface antigens" that are expressed naturally on the surface of a microorganism (*e.g*., a pathogen), or the surface of an infected cell, or the surface of a tumor cell.

As used herein, the term "immunogenic composition" means a two or more immunogens (antigens) that are capable of inducing a protective immune response in a subject being treated, such as a canine or swine or bovine. The protective effects of an immunogenic composition against one or more pathogens are normally achieved by inducing in the animal subject an immune response, either a cell-mediated or a humoral immune response or a combination of both, and preferably a protective immune response.

The term "outer membrane capsular polypeptide immunogen" or "OMC immunogen" refers to a preparation of outer membrane proteins that are isolated or substantially removed from intact Leptospira cells. A representative method for preparing OMC immunogens is provided in, *e.g*., Yang et al., J. Am. Soc. Nephrol. 13:2037-2045 (2002).

The term "protective immunity" as used herein refers to immunity acquired against an immunogen, when a subject has been exposed to the immunogen, which is an immune response (either active/acquired or passive/innate, or both) in the subject that leads to inactivation and/or reduction in the amount of a pathogen and results in immunological memory (*e.g*., memory T- or B-cells). Protective immunity provided by a vaccine can be in the form of humoral immunity (antibody-mediated) or cellular immunity or both. For example, protective immunity can result in a reduction in viral or bacterial shedding, a decrease in incidence or duration of infections, reduced acute phase serum protein levels, reduced rectal temperatures, or increase in food uptake or growth.

As used herein, a "vaccine" is a composition that can be used to elicit protective immunity in a recipient. A subject that has been vaccinated with an immunogen will develop an immune response that prevents, delays, or lessens the development or severity of a disease or disorder in the subject exposed to the immunogen, or a related immunogen, as compared to a non-vaccinated subject. Vaccination may, for example, elicit an immune response that eliminates or reduces the number of pathogens or infected cells, or may produce any other clinically measurable alleviation of an infection.

As used herein, "adjuvant" is to be understood in its broadest sense and includes any immune-stimulating compound. In certain preferred embodiments, the adjuvant is alum, a water-soluble polymer of acrylic acid crosslinked with polyallyl sucrose (*e.g*., Carbopol®), dimethyl dioctadecyl ammonium bromide (DDA), or any combination thereof. Any adjuvant maybe used in conjunction with one or more additional adjuvants. Other exemplary adjuvants include oil-emulsions and emulsifier-based adjuvants such as a water-in-oil emulsion, oil-in-water emulsion, water-in-oil-in-water emulsion, complete Freund's adjuvant, incomplete Freund's adjuvant, MF59, or SAF; mineral gels such as aluminum hydroxide (alum, for example, Al(OH)₃ reHydragel available from Reheis, Berkley Heights, NJ), aluminum salts (*e.g*., aluminum phosphate) or calcium salts (*e.g*., calcium phosphate); microbially-derived adjuvants such as cholera toxin (CT), pertussis toxin, Escherichia coli heat-labile toxin (LT), mutant toxins (*e.g*., LTK63 or LTR72), Bacille Calmette-Guerin (BCG), lipopolysaccharides (LPS), mycobacteria, tetanus toxin, Corynebacterium parvum, DNA CpG motifs, muramyl dipeptide, or monophosphoryl lipid A; particulate adjuvants such as immunostimulatory complexes (ISCOMs), liposomes, biodegradable microspheres, or saponins (*e.g*., QS-21); cytokines such as IFN-γ, IL-2, IL-12 or GM-CSF; synthetic adjuvants such as nonionic block copolymers or surfactants, muramyl peptide analogues (*e.g*., N-acetyl-muramyl-L-threonyl-D-isoglutamine [thr-MDP], N-acetyl-nor-muramyl-L-alanyl-D-isoglutamine, N-acetylmuramyl-L-alanyl-D-isoglutaminyl-L-alanine-2-[1'-2'-dipalmitoyl-sn-glycero-3-hydroxyphosphoryloxy]-ethylamine), polyphosphazenes, synthetic polynucleotides, partial esterified polyacrylic acid with a molecular weight of 450,000 D (Carbopol 907 (PAA), Goodrich, Cleveland, Ohio, U.S.A.; as disclosed in U.S. Patent Nos. 6,340,464 and 6,610,310) and surface active substances, such as lysolecithin, pluronic polyols, polyanions, peptides, hydrocarbon emulsions, or keyhole limpet hemocyanins (KLH). Additional adjuvants include CMC (carboxyl methylcellulose) and HPMC (hydroxypropyl methylcellulose).

As used herein, the terms "serogroup" and "serovar" relate to a classification of Leptospira which is based upon serological typing data, in particular data obtained using agglutination assays, such as the microscopic agglutination test (MAT). An exemplary MAT is set forth in Example 1 herein. For example, "serogroup" or "serovar" refers to a group of Leptospira whose members cross-agglutinate with shared group antigens, but do not cross-agglutinate with the members of other groups and, as a consequence, the members of a serogroup (serovar) have more or less close antigenic relations with one another by simple cross-agglutination. Moreover, Leptospira that belong to different species may be classified in the same serovar or serogroup because they are indistinguishable by antigenic determination.

The term "antibody," as used herein, is intended to include fragments thereof which are also specifically reactive with a molecule which comprises, mimics, or cross-reacts with a B cell or T cell epitope of a surface molecule or surface polypeptide or other molecule produced by a Leptospira serovar, *Bordetella bronciseptica,* or a virus. Antibodies can be fragmented using conventional techniques. For example, F(ab')2 fragments can be generated by treating antibody with pepsin. The resulting F(ab')2 fragment can be treated to reduce disulfide bridges to produce Fab' fragments.

The term "therapeutically effective amount" or "effective amount" refers to an amount of an immunogenic composition sufficient to elicit a protective immune response in the subject (*e.g*., a dog or a pig or a cow) to which it is administered. As noted herein, the immune response may result in an induction of cellular or humoral immunity. The amount of a multivalent immunogen composition that is therapeutically effective may vary depending on the particular antigen used in the composition, the age and condition of the subject being treated, or the degree of infection, if any.

### IMMUNOGENIC COMPOSITIONS

In one aspect, the present disclosure is generally directed to compositions comprising isolated outer membrane capsular (OMC) polypeptide immunogens derived from Leptospira or inactivated whole or partial cell preparations of Leptospira. Accordingly, one embodiment of the present disclosure is directed to an immunogenic composition comprising a Leptospira cell surface immunogen of *Leptospira bratislava, Leptospira autumnalis, Leptospira australis,* and *Leptospira hebdomadis.* In another embodiment, this immunogenic composition having four Leptospira cell surface immunogens, further comprises a *B*. *bronchiseptica* antigen, preferably a p68 antigen. In certain preferred embodiments, the Leptospira cell surface immunogens are OMC immunogens. In other preferred embodiments, the immunogenic composition is capable of eliciting an immune response in a canine, such as a dog, and more preferably a protective immune response.

In certain embodiments, the immunogenic composition is capable of eliciting an immune response in a human or livestock or companion animal, such as swine, bovine, ovine, caprine, equine, deer, vicuña, canine or feline. In a preferred embodiment, the immune response elicited is a protective immune response.

In another aspect of the present disclosure, the immunogen component of an effective composition may further comprise one or more viral immunogens.

The viral immunogen may be a complete, attenuated viral immunogen that has been passaged or pre-treated to render it non-infectious and predominantly asymptomatic. Immunogens that may be employed in the generation of the compositions, including immunogenic compositions described herein, may be live, attenuated (such as CD, CAV-2, CPI, and CPV), or killed (inactivated) virions (such as CCV). If attenuated, then serial passaging of the virus using available technology may be recommended to lessen its virulence, while retaining its immunogenicity. Whole or subunit influenza virions may be inactivated by conventional means such as, for example, through chemical inactivation using one or more chemical inactivating agents including, but not limited to, one or more of binary ethyleneimine, beta-propiolactone, formalin, gluteraldehyde, and/or sodium dodecyl sulfate. Virions may also be inactivated by heat or psoralen in the presence of ultraviolet light. Methods of attenuating virulent strains of these viruses and methods of making an inactivated viral preparation are known in the art and are described in, e.g., U.S. Pat. Nos. 4,567,042 and 4,567,043. Antigens from these pathogens for use in the vaccine compositions of the present invention can be in the form of a modified live viral preparation or an inactivated viral preparation.

In one embodiment, an immunogenic composition of the present disclosure includes attenuated strains of a canine distemper (CD) virus, canine adenovirus type 2 (CAV-2), canine parainfluenza (CPI) virus and canine parvovirus (CPV); an inactivated preparation of canine coronavirus (CCV); and a cell surface immunogen of four Leptospira serovars (*L. bratislava, L. autumnalis, L. australis,* and *L. hebdomadis*). In another embodiment, this immunogenic composition further comprises three additional Leptospira serovars (*L. canicola, L. grippotyphosa,* and *L*. *icterohaemorrhagiae*). In still another embodiment, this immunogenic composition having five viral immunogens and seven Leptospira cell surface immunogens, further comprises a *Bordetella bronchiseptica* antigen, preferably the p68 antigen. In certain preferred embodiments, the Leptospira cell surface immunogens are OMC immunogens. In other preferred embodiments, the immunogenic composition is capable of eliciting an immune response in a canine, such as a dog, and more preferably a protective immune response.

In a further aspect of the present disclosure, an immunogenic composition includes attenuated strains of a CD virus, CAV-2, CPI virus, and CPV; and a cell surface immunogen of four Leptospira serovars (*L. bratislava, L. autumnalis, L. australis,* and *L*. *hebdomadis*). In another embodiment, this immunogenic composition further comprises three additional Leptospira serovars (*L*. *canicola*, *L. grippotyphosa,* and *L. icterohaemorrhagiae*)*.* In still another embodiment, this immunogenic composition having four viral immunogens and seven Leptospira cell surface immunogens, further comprises a *B*. *bronchiseptica* antigen, preferably the p68 antigen. In certain preferred embodiments, the Leptospira cell surface immunogens are OMC immunogens. In other preferred embodiments, the immunogenic composition is capable of eliciting an immune response in a canine, such as a dog, and more preferably a protective immune response.

In still a further aspect of the present disclosure, an immunogenic composition includes attenuated strains of a CD virus, CAV-2, and CPV; and a cell surface immunogen of four Leptospira serovars (*L. bratislava, L. autumnalis, L. australis,* and *L. hebdomadis*)*.* In another embodiment, this immunogenic composition further comprises three additional Leptospira serovars (*L. canicola, L. grippotyphosa,* and *L. icterohaemorrhagiae*). In still another embodiment, this immunogenic composition having three viral immunogens and seven Leptospira cell surface immunogens, further comprises a *B. bronchiseptica* antigen, preferably the p68 antigen. In certain preferred embodiments, the Leptospira cell surface immunogens are OMC immunogens. In other preferred embodiments, the immunogenic composition is capable of eliciting an immune response in a canine, such as a dog, and more preferably a protective immune response.

In yet a further aspect of the present disclosure, an immunogenic composition includes an attenuated strain of a CPI virus; and a cell surface immunogen of four Leptospira serovars (*L. bratislava, L. autumnalis, L. australis,* and *L*. *hebdomadis*). In another embodiment, this immunogenic composition further comprises three additional Leptospira serovars (*L*. *canicola, L. grippotyphosa,* and *L*. *icterohaemorrhagiae*). In still another embodiment, this immunogenic composition having one viral immunogen and seven Leptospira cell surface immunogens, further comprises a *B. bronchiseptica* antigen, preferably the p68 antigen. In certain preferred embodiments, the Leptospira cell surface immunogens are OMC immunogens. In other preferred embodiments, the immunogenic composition is capable of eliciting an immune response in a canine, such as a dog, and more preferably a protective immune response.

In certain embodiments of the present disclosure, an immunogenic composition includes attenuated strains of a CD virus, CAV-2, CPI virus, and CPV; an inactivated preparation of CCV; and a cell surface immunogen of three Leptospira serovars (*L. autumnalis, L. australis,* and *L*. *hebdomadis*). In another embodiment, this immunogenic composition further comprises two additional Leptospira serovars (*L. canicola* and *L*. icterohaemorrhagiae). In still another embodiment, this immunogenic composition having five viral immunogens and five Leptospira cell surface immunogens, further comprises a *B. bronchiseptica* antigen, preferably the p68 antigen. In certain preferred embodiments, the Leptospira cell surface immunogens are OMC immunogens. In other preferred embodiments, the immunogenic composition is capable of eliciting an immune response in a canine, such as a dog, and more preferably a protective immune response.

In further embodiments of the present disclosure, an immunogenic composition includes attenuated strains of a CD virus, CAV-2, CPI virus, and CPV; an inactivated preparation of CCV; and a cell surface immunogen of five Leptospira serovars (*L. bratislava, L. canicola, L. icterohaemorrhagiae, L. grippotyphosa,* and *L*. *pomona).* In another embodiment, this immunogenic composition having five viral immunogens and five Leptospira cell surface immunogens, further comprises a *B*. *bronchiseptica* antigen, preferably the p68 antigen. In certain preferred embodiments, the Leptospira cell surface immunogens are OMC immunogens. In other preferred embodiments, the immunogenic composition is capable of eliciting an immune response in a swine or canine, such as a pig or dog, respectively, and more preferably a protective immune response.

In yet a further embodiment of the present disclosure, an immunogenic composition includes attenuated strains of a CD virus, CAV-2, CPI virus, and CPV; an inactivated preparation of CCV; and a cell surface immunogen of four Leptospira serovars (*L*. *canicola, L. icterohaemorrhagiae, L. grippotyphosa,* and *L*. *pomona*)*.* In another embodiment, this immunogenic composition having five viral immunogens and four Leptospira cell surface immunogens, further comprises a *B*. *bronchiseptica* antigen, preferably the p68 antigen. In certain preferred embodiments, the Leptospira cell surface immunogens are OMC immunogens. In other preferred embodiments, the immunogenic composition is capable of eliciting an immune response in a canine, such as a dog, and more preferably a protective immune response.

In another aspect of the present disclosure, an immunogenic composition includes attenuated strains of a CD virus, CAV-2, CPI virus, and CPV; an inactivated preparation of CCV; and a cell surface immunogen of four Leptospira serovars (*L*. *bratislava, L. icterohaemorrhagiae, L. grippotyphosa,* and *L*. *pomona*)*.* In certain preferred embodiments, the Leptospira cell surface immunogens are OMC immunogens. In other preferred embodiments, the immunogenic composition is capable of eliciting an immune response in a swine, such as a pig, and more preferably a protective immune response.

In still another aspect of the present disclosure, an immunogenic composition includes attenuated strains of a CD virus, CAV-2, CPI virus, and CPV; an inactivated preparation of CCV; and a cell surface immunogen of five Leptospira. serovars (*L*. *canicola, L. hardjo, L. icterohaemorrhagiae, L. grippotyphosa,* and *L*. *pomona).* In certain preferred embodiments, the Leptospira cell surface immunogens are OMC immunogens. In other preferred embodiments, the immunogenic composition is capable of eliciting an immune response in a bovine, such as a cow, and more preferably a protective immune response.

In other embodiments of the present disclosure, an immunogenic composition includes attenuated strains of a CD virus, CAV-2, CPI virus, and CPV; an inactivated preparation of CCV; and a *B*. *bronchiseptica* antigen, preferably the p68 antigen. In other preferred embodiments, the immunogenic composition is capable of eliciting an immune response in a canine, such as a dog, and more preferably a protective immune response.

The present disclosure also includes any of the foregoing immunogenic compositions, wherein a *B. bronchiseptica* antigen is specifically excluded from the compositions. For example, an immunogenic composition comprising a viral immunogen of an attenuated strain of canine distemper (CD) virus, an attenuated strain of canine adenovirus type 2 (CAV-2), an attenuated strain of canine parainfluenza (CPI) virus, an attenuated strain of canine parvovirus (CPV), an inactivated or partial cell preparation of a strain of canine coronavirus (CCV) and an *L. bratislava* antigen (*e.g*., an L. bratislava outer membrane capsular (OMC) protein), wherein the composition does not include a *B. bronchiseptica* antigen.

Exemplary immunogenic compositions are also provided in Table 1:

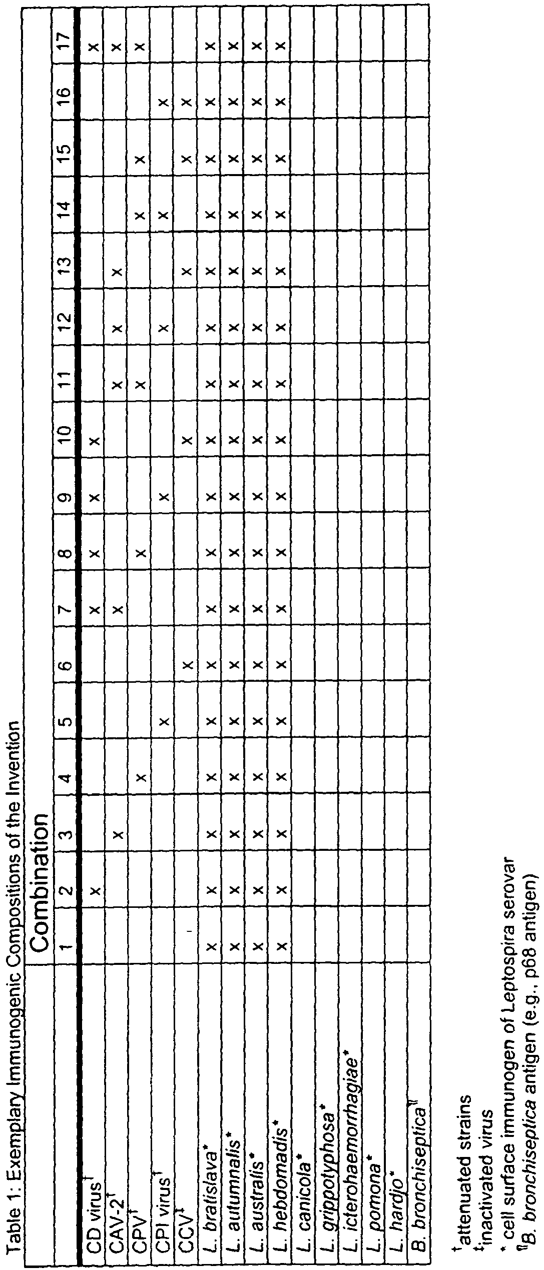

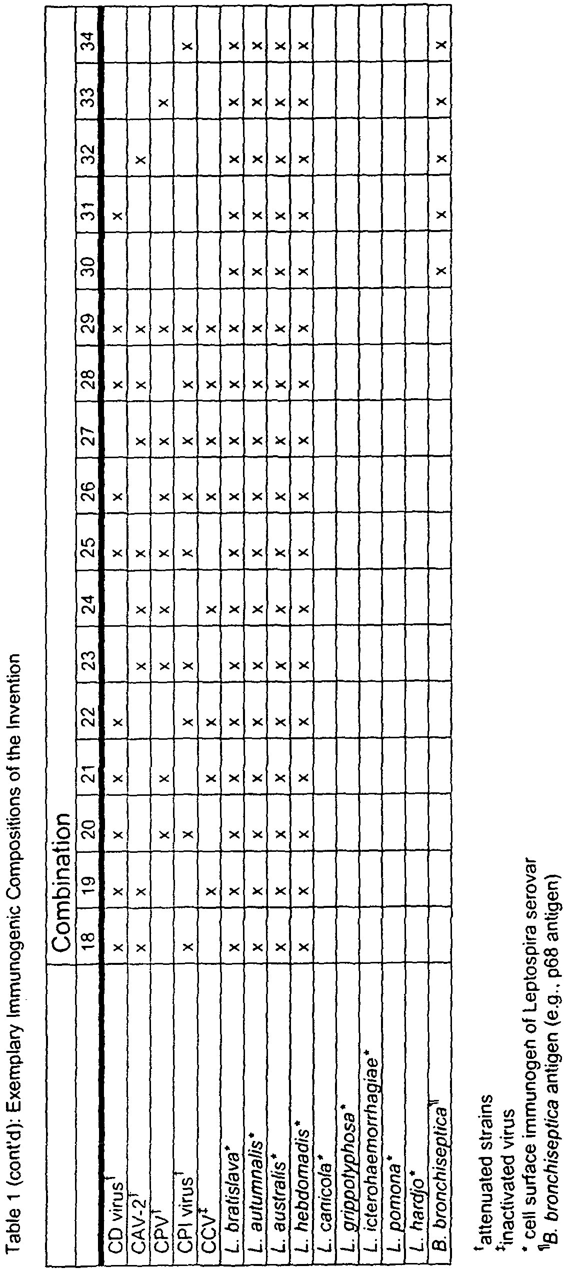

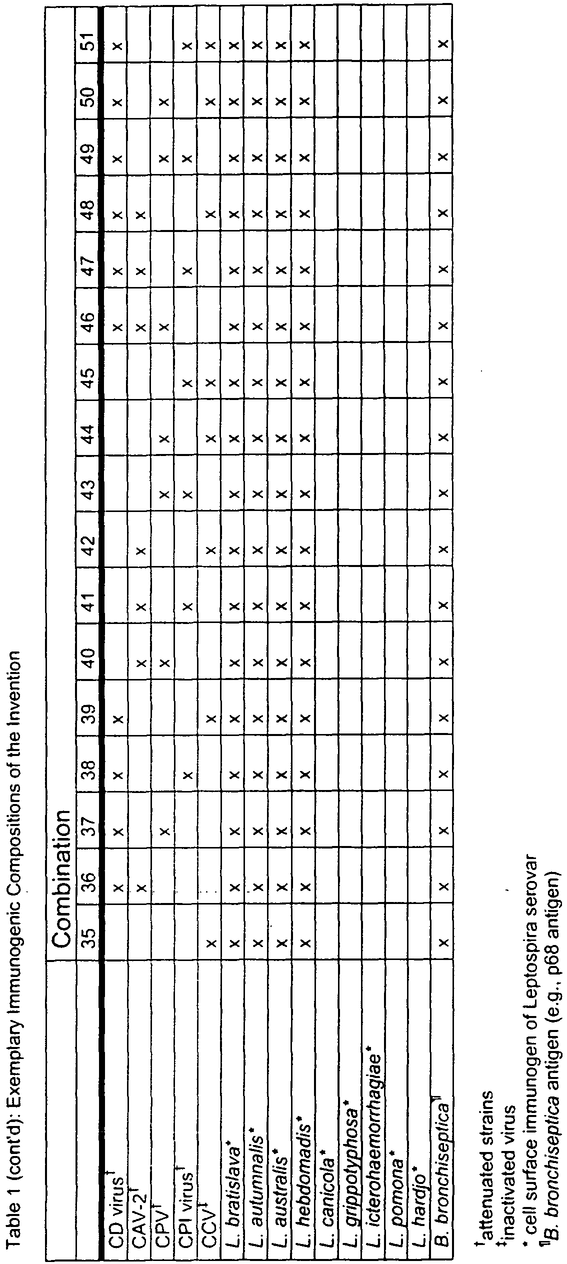

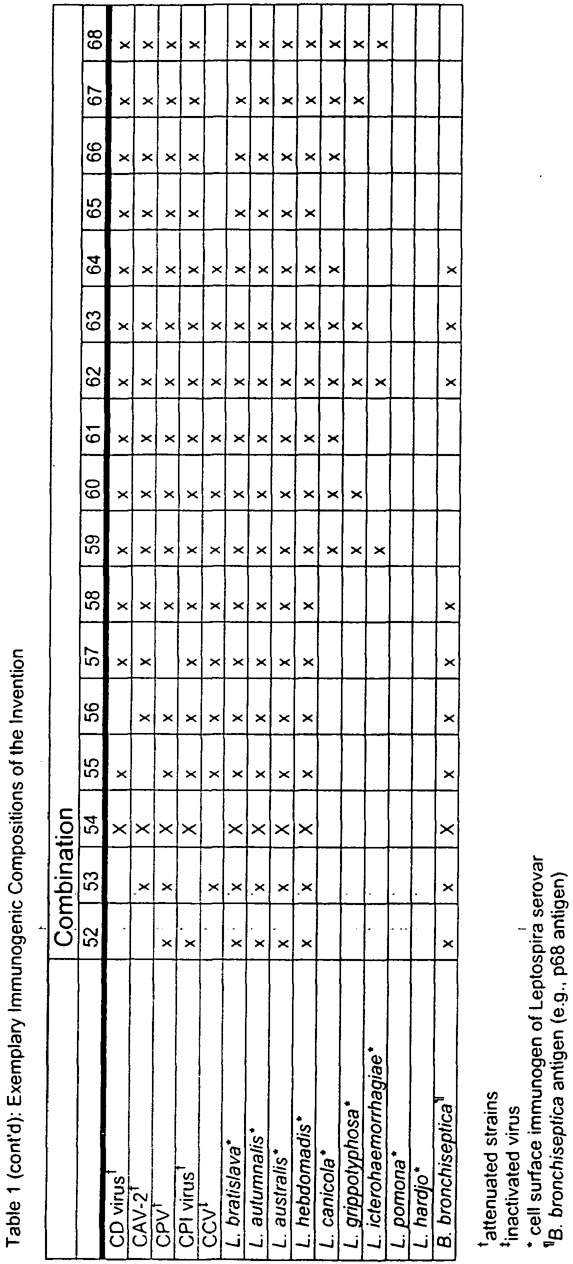

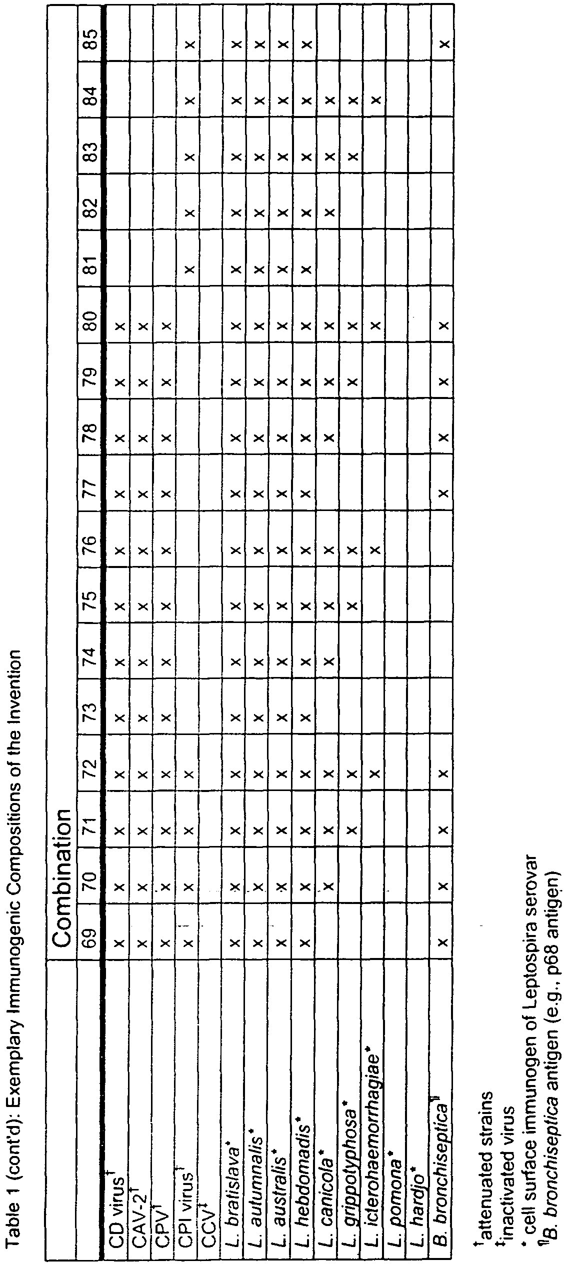

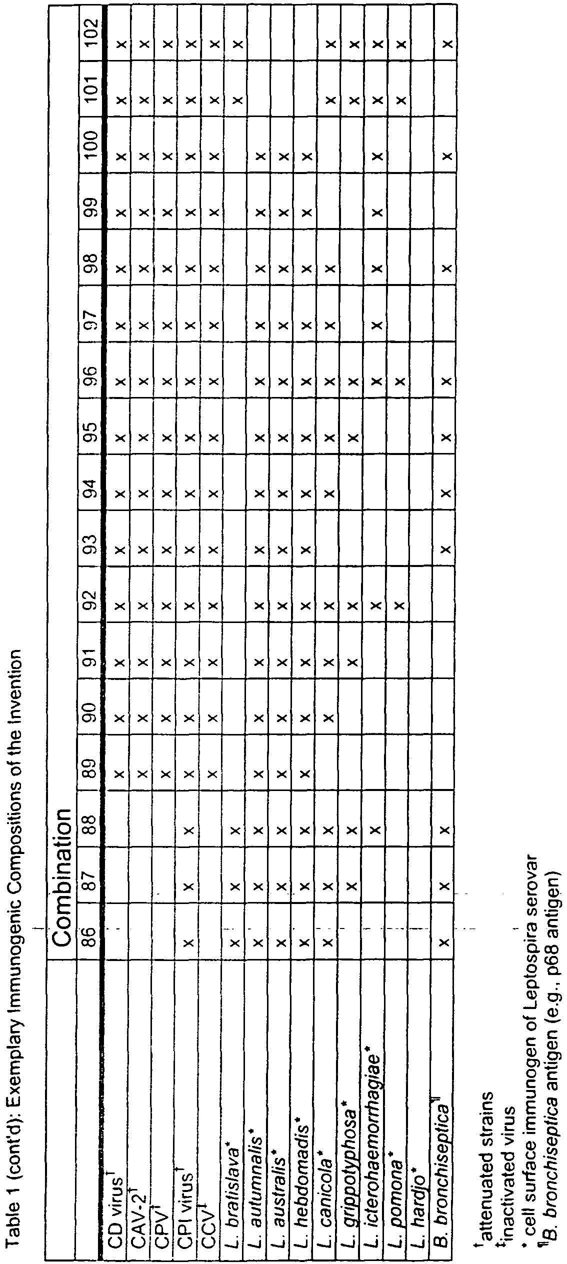

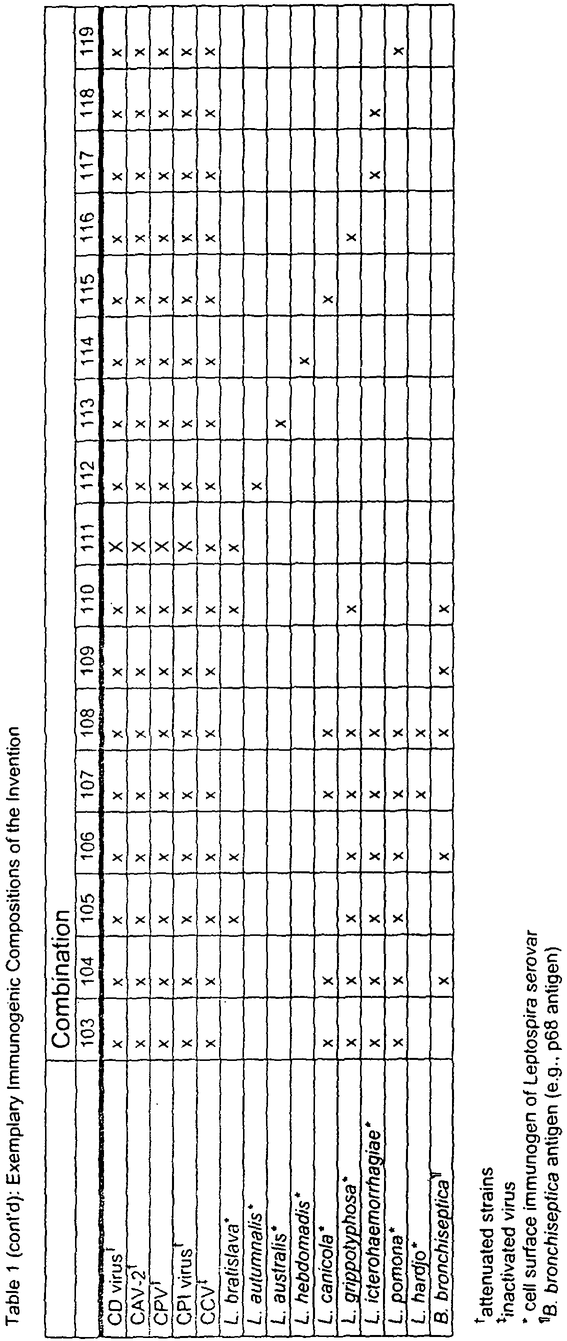

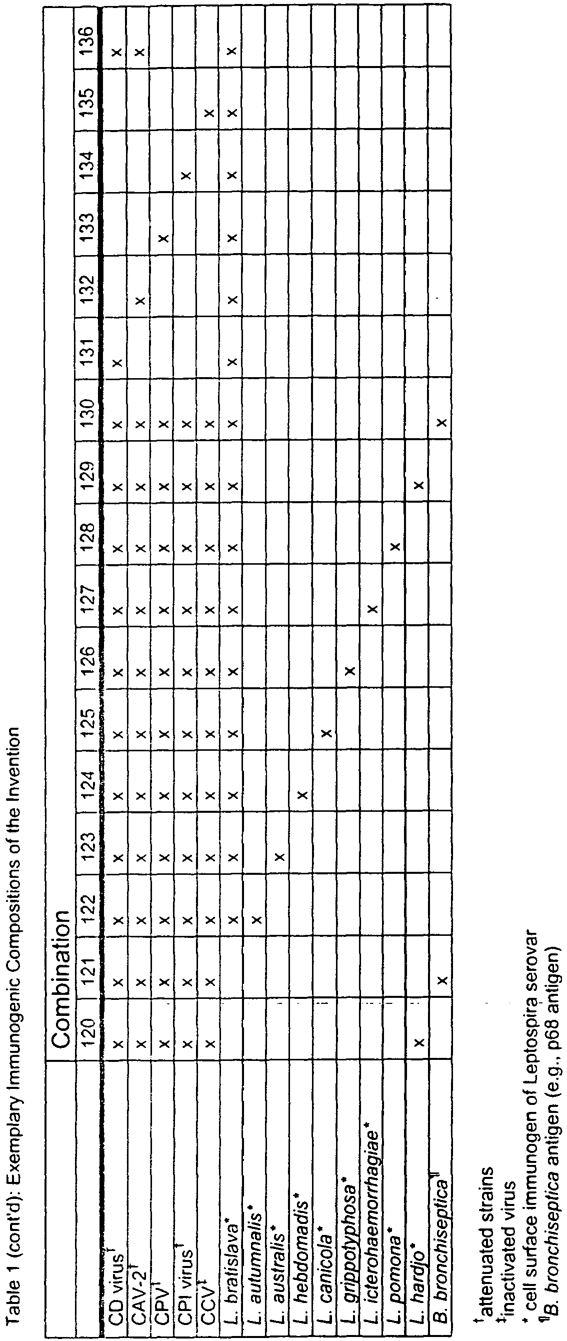

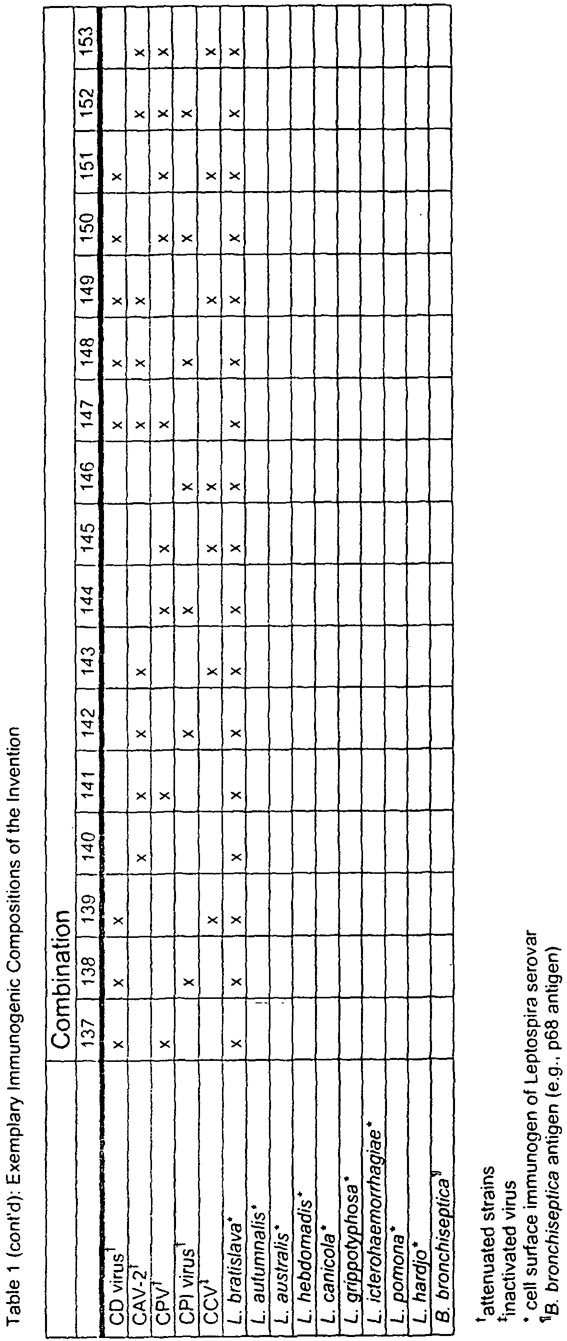

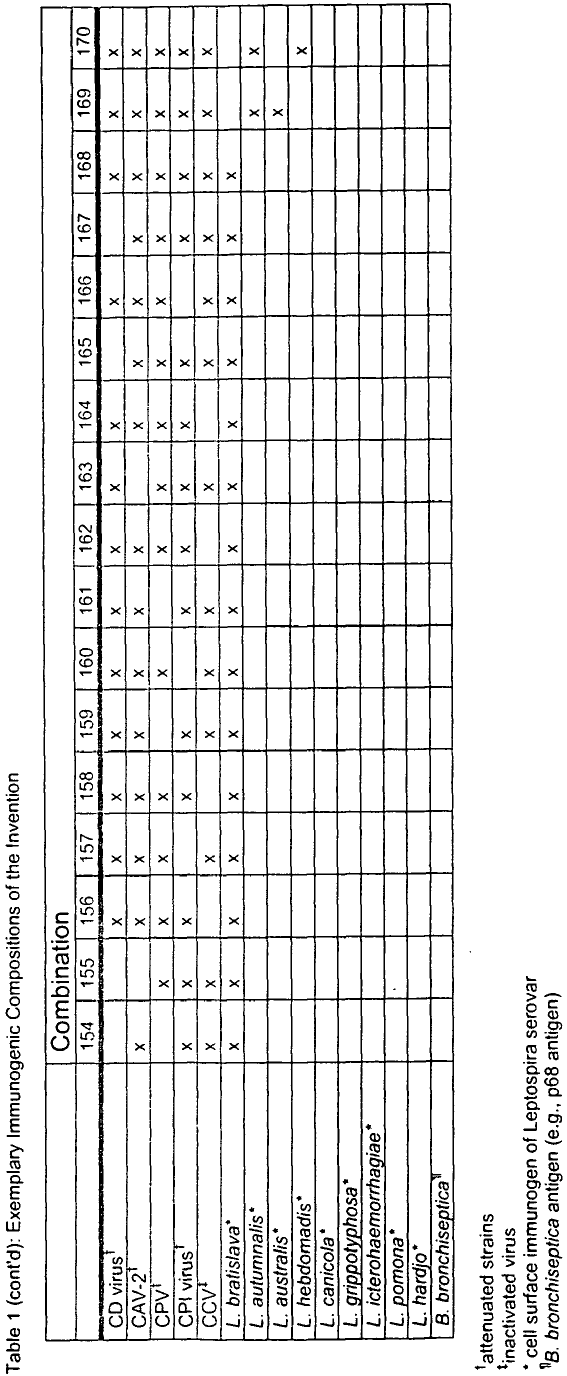

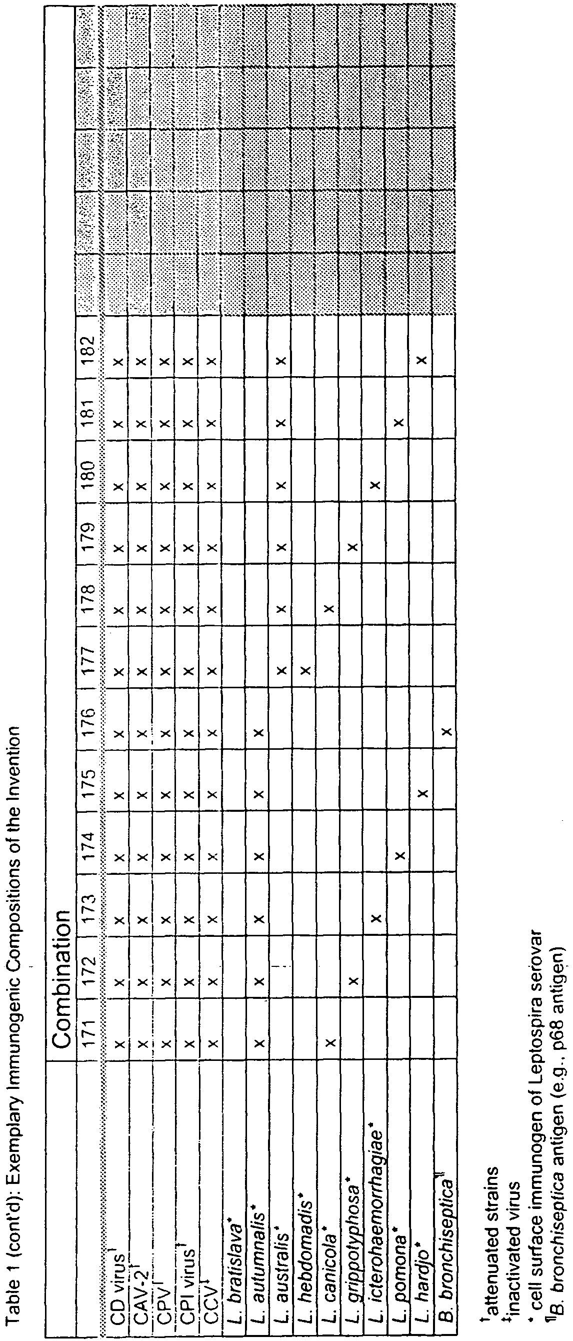

The exemplary combinations set forth in Table 1 above are intended to be non-limiting. For example, Combination 1 set forth in Table 1 is meant to include immunogenic compositions that contain antigens from *L. bratislava, L. autumnalis, L. australis,* and *L. hebdomadis,* has well as other immunogenic components not explicitly listed in the Table. In addition. Combination 1 is also, intended to denote an immunogenic composition that contains antigens from *L*. *bratislava, L. autumnalis, L. australis,* and *L*. *hebdomadis*, and specifically does not include one or more of the immunogenic componenis listed in Table 1 that are not marked with an 'x' under Combination 1.

In view of the teachings set forth herein, additional combinations can be easily made and lested by persons of ordinary skill in the art

Any of the immunogenic composition embodiments described in the instant disclosure may further have one or more pharmaceutically- or veterinary-acceptable carriers or diluents. In other embodiments, the immunogenic compositions may further comprise at least one adjuvant or at least one preservative or any combination thereof. Preferably, the immunogenic compositions according to these embodiments are a vaccine preparation. In certain embodiments, the compositions induce a humoral immune response against one or more Leptospira serovar, one or more viral immunogen, or a *B*. *bronchiseptica* antigen when administered to a human or animal subject, preferably a canine, swine or bovine.

Still another preferred combination vaccine includes an attenuated strain of CD virus, an attenuated strain of CAV-2, an attenuated strain of CPI virus, an attenuated strain of CPV, and a recombinant Bordetella bronchiseptica p68 antigen.

### FORMULATIONS AND USES

As set forth herein, the present disclosure provides an immunogenic composition suitable for administration to a subject, such as a canine, swine, or bovine, to elicit an immune response. The immunogenic composition of the present disclosure can further include a *Bordetella bronchiseptica* antigen, such as p68 or recombinant p68, which composition is capable of inducing an immune response in, for example, canine, swine or bovine. Another embodiment of the present disclosure includes a composition comprising two or more antigens from canine pathogens, capable of inducing an immune response in dogs against disease caused by such pathogen(s).

The immunogenic compositions of the present disclosure are further contemplated to exhibit excellent therapeutic activity, for example, in the prevention of infection by one or more Leptospira pathogens, one or more viral pathogens, or *Bordetella bronchiseptica.* Preferably, the multivalent immunogen composition is effective in mediating a protective immune response when administered to an animal, such as to a human or livestock or a companion animal, such as swine, bovine, ovine, caprine, equine, deer, vicuña, canine, feline, or the like.

The composition may be administered in a convenient manner such as by the oral, intravenous (where water soluble), intramuscular, subcutaneous, intranasal, intradermal or suppository routes or by implantation (*e.g*., using slow release molecules). Depending on the route of administration, the immunogens contained therein may be required to be coated in a material to protect them from the action of enzymes, acids and other natural conditions which otherwise might inactivate the immunogens. In order to administer the composition by other than parenteral administration, they will be coated by, or administered with, a material to prevent its inactivation. For example, the immunogen may be administered with an adjuvant, co-administered with enzyme inhibitors, or in liposomes.

The composition of the present invention may also be administered parenterally or intraperitoneally. Dispersions of the immunogen component can also be prepared in glycerol, liquid polyethylene glycols, and mixtures thereof and in oils. Under ordinary conditions of storage and use, these preparations may contain a preservative to prevent the growth of microorganisms, such a gentamycin.

As used herein "pharmaceutically acceptable or veterinary-acceptable carrier and/or diluent" includes any and all solvents, dispersion media, coatings, antibacterial and antifungal agents, isotonic and absorption delaying agents and the like. The use of such media and agents for pharmaceutically active substances is well-known in the art. Supplementary active ingredients, such as antimicrobials, can also be incorporated into the compositions.

The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be effected by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the multivalent immunogen compositions of the present disclosure in the required amount of the appropriate solvent with various of the other ingredients enumerated herein, as required, followed by heat-sterilization, irradiation or other suitable sterilization means. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutically or veterinary acceptable carrier.

The present disclosure also provides methods of protecting a subject, such as canines, swine, or bovines, against bacterial or viral infections by administering a multivalent immunogen composition as described herein.

In a further embodiment, the disclosure provides an immunogenic composition, as described herein, further containing a *Bordetella bronchiseptica* p68 antigen, which is suitable for administration to, for example, dogs, and is capable of protecting dogs against disease caused by *Bordetella bronchiseptica, e.g.,* infectious tracheobronchitis ("kennel cough").

In accordance with the present invention, p68 antigens suitable for use in the present invention include both native p68 proteins (*i.e*., naturally occurring p68 proteins purified from *Bordetella bronchiseptica*) and recombinantly produced p68 proteins. Purification of native p68 from *Bordetella bronchiseptica* is known in the art (see, *e.g.,* Montaraz et al., Infect. Immun. 47: 744-751, 1985, and U.S. Pat. Pub. No. 2004/0185062). Recombinant production of p68 can be achieved using any one of the molecular cloning and recombinant expression techniques known to those skilled in the art. For example, a nucleic acid molecule encoding p68 can be introduced into an appropriate host cell, such as a bacterium, a yeast cell (*e.g*., a Pichia cell), an insect cell or a mammalian cell (*e.g*., CHO cell).

Preferably, the immunogenic compositions are formulated to be administered by injection at a unit dosage volume of about 0.1 to 5 ml, preferably at a unit dosage volume of about 0.5 to about 2.5 ml, or even more preferably at a unit dosage volume of about 1 ml. In certain embodiments, the pharmaceutical compositions of the present invention should be made sterile by well-known procedures.

The amount of the *B*. *bronchiseptica* antigen in the immunogenic compositions should be effective at eliciting an immune response and is generally provided in the range of about 0.5 to about 1000 µg per dose. Preferably, the amount of p68 antigen is in the range of about 1 µg to about 260 µg per dose. More preferably, the amount of p68 antigen is in the range of about 10 µg to about 100 µg per dose. Even more preferably, the amount of p68 antigen is about 15 to about 25 µg per dose.

Pharmaceutical compositions comprising the immunogens of the disclosure may be manufactured by means of conventional mixing, dissolving, granulating, dragee making, levigating, emulsifying, encapsulating, entrapping or lyophilizing processes. Pharmaceutical compositions may be formulated in conventional manner using one or more physiologically acceptable carriers, diluents, excipients or auxiliaries that facilitate formulating active antimicrobial lipopeptide derivatives into preparations that can be used pharmaceutically. The multivalent immunogen compositions can be combined with one or more biologically active agents and may be formulated with a pharmaceutically acceptable carrier, diluent or excipient to generate pharmaceutical or veterinary compositions of the instant disclosure.

Pharmaceutically acceptable carriers, diluents or excipients for therapeutic use are well known in the pharmaceutical art, and are described herein and, for example, in Remington's Pharmaceutical Sciences, Mack Publishing Co. (A.R. Gennaro, ed., 18th Edition, 1990) and in CRC Handbook of Food, Drug, and Cosmetic Excipients, CRC Press LLC (S.C. Smolinski, ed., 1992). In certain embodiments, immunogenic compositions may be formulated with a pharmaceutically or veterinary-acceptable carrier, diluent or excipient is aqueous, such as water or a mannitol solution (*e.g*., about 1% to about 20%), hydrophobic (*e.g*., oil or lipid), or a combination thereof (*e.g*., oil and water emulsions). In certain embodiments, any of the pharmaceutical compositions described herein have a preservative or stabilizer (*e.g*., an antibiotic) or are sterile.

The pharmaceutical compositions of the present disclosure can be formulated to allow the immunogens contained therein to be bioavailable upon administration of the composition to a subject. The level of immunogen in serum and other tissues after administration can be monitored by various well-established techniques, such as chromatographic or antibody (*e.g*., ELISA) based assays. In other embodiments, immunogenic compositions are formulated for parenteral administration to a subject in need thereof (*e.g*., having Gram-positive bacterial infection), such as an animal or a human. Preferred routes of administration include subcutaneous and intramuscular administrations.

Proper formulation is dependent upon the route of administration chosen, as is known in the art. For example, systemic formulations are an embodiment that includes those designed for administration by injection, *e.g*. subcutaneous, intravenous, intramuscular, intrathecal or intraperitoneal injection, as well as those designed for transdermal, transmucosal, oral, intranasal, or pulmonary administration. In one embodiment, the systemic formulation is sterile. In embodiments for injection, the immunogenic compositions of the instant disclosure may be formulated in aqueous solutions, preferably in physiologically compatible solutions or buffers such as Hanks's solution, Ringer's solution, mannitol solutions or physiological saline buffer. In certain embodiments, any of the immunogenic compositions described herein may contain formulatory agents, such as suspending, stabilizing or dispersing agents. Alternatively, the immunogenic compositions may be in solid (*e.g*., powder) form for constitution with a suitable vehicle (*e.g*., sterile pyrogen-free water) before use. In embodiments for transmucosal administration, penetrants, solubilizers or emollients appropriate to the barrier to be permeated may be used in the formulation. For example, 1-dodecylhexahydro-2H-azepin-2-one (Azone®), oleic acid, propylene glycol, menthol, diethyleneglycol ethoxyglycol monoethyl ether (Transcutol®), polysorbate polyethylenesorbitan monolaurate (Tween®-20), and the drug 7-chloro-1-methyl-5-phenyl-3H-1,4-benzodiazepin-2-one (Diazepam), isopropyl myristate, and other such penetrants, solubilizers or emollients generally known in the art may be used in any of the compositions of the instant disclosure. Administration can be achieved using a combination of routes, e.g., first administration using a parental route and subsequent administration using a mucosal route.

In another aspect, the present disclosure provides methods of protecting a subject against bacterial or viral infections, as described herein. In accordance with the present disclosure, the immunogenic composition provides dogs with a long term immunity for at least about 4 months, preferably for at least about 6 months, or even more preferably for about one year.

The immunogenic composition of the present invention can be administered to dogs that are at least 6 weeks old, preferably at least 7 weeks old, and more preferably, at least 8 or 9 weeks old. Dogs can be vaccinated with one dose or with more than one dose of an immunogenic composition. Preferably, two doses of an immunogenic composition are administered to dogs with an interval of about 2-4 weeks, preferably about 3 weeks, between the two administrations. If dogs are vaccinated before the age of 4 months, it is recommended that they be revaccinated with a single dose upon reaching 4 months of age, because maternal antibodies may interfere with development of an adequate immune response in puppies less than 4 months old. Dogs can also be revaccinated annually with a single dose. When *B*. *bronchiseptica* exposure is likely, such as breeding, boarding, and showing situations, an additional booster may be given within 1 year, or preferably 6 months, of the occurrence of these events.

In accordance with the present disclosure, the immunogenic compositions generally include a veterinary-acceptable carrier. As described herein, a veterinary-acceptable carrier includes any and all solvents, dispersion media, coatings, adjuvants, stabilizing agents, diluents, preservatives, antibacterial and antifungal agents, isotonic agents, adsorption delaying agents, and the like. Diluents can include water, saline, dextrose, ethanol, glycerol, and the like. Isotonic agents can include sodium chloride, dextrose, mannitol, sorbitol, and lactose, among others. Stabilizers include albumin, among others.

Preferably, the immunogenic composition is formulated such that it can be administered to a subject, such as a dog, by injection in a dose of about 0.1 to 5 ml, or preferably about 0.5 to 2.5 ml, or even more preferably, in a dose of about 1 ml.

Immunogenic compositions may be prepared by rehydrating a freeze-dried preparation of the attenuated viral strains (or a preparation made by other methods such as spray drying or desiccation) and viral preparation with a liquid preparation, which liquid preparation is composed of the Leptospiral antigens, dissolved in sterile saline solution and adjuvanted. Such immunogenic compositions may also be prepared by rehydrating a freeze-dried preparation of the attenuated viral strains and Leptospira viral preparation (or a preparation made by other methods such as spray drying or desiccation) with a sterile solution, or rehydrating the freeze-dried preparation with CCV plus diluent.

In accordance with the present invention, immunogenic compositions can be administered to a dog of at least 6 weeks old, preferably at least 7 weeks old, and more preferably, at least 8 or 9 weeks old. The combination vaccines can be administered in 2 to 4 doses, preferably in 2 to 3 doses. The doses can be administered with 2 to 6 weeks between each dose, preferably with 2 to 4 weeks between each dose.

The attenuated CD virus should be in an amount of at least about 10² to about 10⁹ TCID₅₀ (tissue culture infectious dose 50% cytopathic effect) per dose, and preferably in the range of about 10⁴ to about 10⁶ TCID₅₀ per dose. The attenuated CAV-2 should be in an amount of at least about 10² TCID₅₀ to about 10⁹ TCID₅₀ per dose, preferably in the range of 10⁴ to about 10⁶ TCID₅₀ per dose. The attenuated CPI virus should be in an amount of at least about 10² TCID₅₀ to about 10⁹ TCID₅₀ per dose, and preferably in the range of 10⁶ to about 10⁸ TCID₅₀ per dose. The attenuated CPV should be in an amount of at least about 10² TCID₅₀ to about 10⁹ TCID₅₀ per dose, preferably, an amount in the range of 10⁷ to about 10⁹ TCID₅₀ per dose. The amount of CCV in an inactivated viral preparation should be at least about 100 relative units per dose, and preferably in the range of 1000-4500 relative units per dose. Each Leptospira species in the vaccine should be in the range of about 100-3500 NU (nephelometric units) per vaccine dose, and preferably in the range of 200-2000 NU per dose. In certain embodiments, dosage units comprise at least about 1 x 10⁶ TCID₅₀, or at least about 1x10⁷ TCID₅₀ or at least about 5x10⁷ TCID₅₀ of killed or inactivated whole or subunit virion or portion thereof. In certain aspects of these embodiments dosage units may comprise as much as 1 X 10⁹ TCID₅₀ or more of killed or inactivated whole or subunit virion or portion thereof. Thus, a suitable range of killed or inactivated whole or subunit virion or portion thereof is between about 1x10⁴ TCID₅₀ and about 1x10⁹ TCID₅₀.

In another aspect, the instant disclosure provides a pharmaceutical kit of parts comprising an immunogenic composition as defined herein. The kit may also further comprise instructions for use in the treatment of a canine, swine, or bovine disease (such as leptospirosis, as described herein). The active agents of the immunogenic composition may be co-packaged in unit dosage form.

The following non-limiting examples are provided to illustrate various aspects of the present disclosure.

### EXAMPLES

### EXAMPLE 1

### IMMUNOGENICITY/EFFICACY OF LEPTOSPIRA BRATlSLAVA IN A COMBINATION VACCINE

### INTRODUCTION

This example illustrates the immunogenicity of *L. bratislava* and lack of antigen blockage when *L. bratislava* is added to a combination vaccine.

DA₂PP/CvK/LCIGP is the designation given to a combination vaccine product containing attenuated canine distemper virus (CD virus), attenuated canine adenovirus type 2 (CAV-2), inactivated canine coronavirus (CCV), attenuated canine parainfluenza virus (CPI virus), and attenuated canine parvovirus (CPV). Outer membrane capsular (OMC) protein derived from *L. bratislava* was added to DA₂PP/CvK/LCIGP to produce a new combination product designated DA₂PP/CvK/LCIGPB.

As shown in this example, *L. bratislava* OMC protein, when blended with DA₂PP/CvK/LCIGP, was effective in inducing protective immunity in puppies against a virulent *L. bratislava* challenge.

### MATERIALS AND METHODS

### ANIMAL SELECTION

### TEST ANIMALS

A total of 34 beagles (22 vaccinates and 12 controls), six-weeks to six-weeks and 6 days old, were utilized in this study. All puppies were purchased from Harlan Sprague Dawley (Indianapolis, IN) and tested negative to the fractions in the combination vaccine. Based on the age range, the 34 puppies were split into two batches for vaccination. Puppies from each batch were randomized into vaccinate and control groups using the random number generator of Microsoft Excel. All vaccinates were vaccinated twice with a 21 day interval. To ensure all puppies were challenged simultaneously, the puppies of the first batch were challenged at 28 days post-second vaccination (DPV2) while the second batch of puppies were challenged at 21 DPV2.

### HOUSING AND CARE OF ANIMALS

Animals were caged until the termination of the study. The space of housing was in compliance with applicable regulations of animal welfare.

### TEST VACCINE

### COMPOSITION OF VACCINE

*L. bratislava* OMC protein was added to the combination product designated CvK/LCIGP (containing canine coronavirus (inactivated), *Leptospira canicola, Leptospira icterohaemorrhagiae, Leptospira grippotyphosa,* and *Leptospira Pomona*) to produce a new combination product designated CvK/LCIGPB. The cake, DA₂PP containing canine distemper virus, canine adenovirus type 2, canine parainfluenza virus and canine parvovirus was combined with CvK/LCIGPB to produce a new combination package product designated DA₂PP/CvK/LCIGPB. The concentration of *L. bratislava* OMC protein in the final combination product was 50 µg/1 ml dose.

### STORAGE

The vaccines were stored between 2°C and 7°C.

### EXPERIMENTAL DESIGN

Puppies from each litter were randomized, according to litter, into two groups as shown in Table 2 below using Microsoft Excel random number generator, then sorted ascendingly:

**TABLE 2: Experimental Design**

| **Group** | **Vaccine** | **Challenge Route with *L. bratislava*** | **Number of Animals** |
|---|---|---|---|
| Principal | DA₂PP/CvK/LCIGPB | Intraperitoneal | 22 |
| Controls | None | Intraperitoneal | 12 |

### VACCINATION

Each animal in the principal group was given two doses (1 mL/dose) of the assigned vaccine subcutaneously on opposite sides of the neck three weeks apart.

Coded vaccines and samples were used to ensure blinding of the study.

### EXPERIMENTAL CHALLENGE

The *L*. *bratislava* challenge strain was strain B26 P2 (supplied by the Center Veterinary Biologics Laboratory, Ames, IA). To obtain enough quantity and good viability, the challenge organisms were cultured in T-80 medium (Sulzer and Jones, U.S. Dept. of Health, Education and Welfare, Publ. No. (CDC) 76-8275, pp. 12-15, Center for Disease Control, Atlanta, GA) at 28(±2)°C. Immediately prior to challenge, the motile organisms were counted and the number of organisms adjusted to 1 x 10¹⁰ organism per dose for challenge. The puppies in the principal and control groups were challenged, via the intraperitoneal (IP) route, with virulent *L*. *bratislava.* The volume of challenge material was adjusted to a 2.0 mL challenge dose.

### OBSERVATIONS AND SAMPLE COLLECTION

### PRE-CHALLENGE OBSERVATION

Rectal temperatures and clinical signs were monitored and recorded for three consecutive days prior to challenge in order to ensure healthy condition.

### POST-CHALLENGE OBSERVATION

After challenge, animals were monitored daily for rectal temperatures and observed for any possible clinical signs associated with *L. bratislava* infection for two weeks and the results recorded.

The puppies were euthanized when the study was terminated and the liver and kidneys were examined for potential gross lesions associated with *L*. *bratislava* infection.

### HEMATOLOGICAL PROFILE

After challenge, anti-coagulated blood samples (2 mL) were collected daily from each puppy for monitoring of total leukocyte count until 15 DPC, except 13 and 14 DPC when the Cell-Dyne (blood counter) was unavailable.

Thrombocytes and differential leukocytes were counted by using the Abbott Cell-Dyne blood counter or an automated cell counter.

### ISOLATION OF L. BRATISLAVA FROM BLOOD SAMPLES

Blood samples were used to inoculate T-80 semi-solid *Leptospira* growth medium. Inoculated tubes were incubated at 28(±2)°C for a period of four weeks. All negative cultures were subcultured in fresh medium and incubated for an additional period of two more weeks before discarding as negative. All cultures were examined weekly using a dark field microscope for the presence of Leptospiras and the results recorded.

### MICRO-AGGLUTINATION TESTS (MAT)

Serum agglutinating antibody to *Leptospira* whole cell antigen was measured using the micro-agglutination assay. Briefly, serial two-fold dilutions of sera were prepared in flat-bottom microtiter plates. To each diluted serum, an equal volume (0.05 mL) of live *L. icterohaemorrhagiae, L. canicola, L. grippotophysa, L. bratislava* or *L. pomona* suspension (equivalent to a nephelometer reading of 20) was added. Proper controls were included on each plate. The plates were shaken for 30 seconds and incubated at room temperature for two hours before the results were recorded. The titer of each serum was defined as the reciprocal of the highest dilution of serum that produced at least 50 percent agglutination.

### DATA ANALYSIS

The experimental unit was the individual animal. The primary outcomes were spirochetemia and antibody titers. The percentages of spirochetemia and antibody titers for animals in each group were calculated.

### RESULTS AND DISCUSSION

### L. BRATISLAVA ANTIGEN LEVEL

Fifty micrograms (50 µg) per dose of *L. bratislava* outer membrane capsular (OMC) protein was loaded in the immunogenicity vaccine.

### SEROLOGICAL RESPONSES

The antibody titers of individual puppies were recorded and analyzed. On 0DPV1, all puppies were negative with antibody titers less than 2. With the exception of one vaccinated puppy, all other vaccinates were seropositive to *L*. *bratislava* by 0DPV2 with geometric mean titer (GMT) of 17. Like other *leptospira* serovars, the GMT increased to 741 at 7DPV2, the peak titer after two vaccinations, and then gradually declined to 405 and 222 at 14 and 21 DPV2, respectively. The antibody titers increased substantially at 7 DPC in both control and vaccinated groups. These results indicate a strong boosting effect in the vaccinates and immunoresponse of the controls. All 12 controls remained negative until 21 DPV2, the day of challenge. These data showed that the *L. bratislava* fraction in the combination product, DA₂PP/CvK/LCIGPB is highly immunogenic in inducing serological immune responses *in vivo.*

### CLINICAL OBSERVATIONS

Rectal temperatures, clinical signs, white blood cell counts and platelet counts were assessed for all puppies. One puppy had one day of ocular discharge. Another puppy had one day of depression/lethargy. Two other puppies had one day of diarrhea, and one puppy had one day of conjunctivitis. The other puppies remained clinically normal after the virulent challenge. None of the puppies had elevated or decreased rectal temperatures, white blood cell counts or platelet counts during the study. Like other canine leptospiras, such as canine *Leptospira canicola, pomona* and *L. ictrohaemorrhagiae,* etc., *L. bratislava* alone causes minimal clinical disease in specific pathogen free dogs evidenced by spirochetemia. However, when it is complicated with other pathogens, stress, immunocompromised conditions, and/or poor field environment, it is likely to cause more significant or even lethal impacts on canine health as is the case with the longer recognized canine leptospirosis serovars. There were no gross lesions observed in the kidney and liver upon the conclusion of the study.

### ISOLATION OF L. BRATISLAVA

The spirochetemia following virulent *L. bratislava* challenge was measured by whole blood culture in T80 medium. With the exception of one puppy, all of the control puppies (11 out of 12 puppies or 92% control puppies) were positive for *L. bratislava.* In contrast, only one puppy in the vaccinated group was positive with spirochetemia. In other words, about 95% (21 out of 22 puppies) of the vaccinates remained free of spirochetemia after virulent *L. bratislava* challenge. These data demonstrate the vaccine is immunologically potent in inducing protective immunity and protecting puppies from replication of the virulent *L. bratislava* post challenge. The results clearly showed that *L. bratislava* outer membrane antigen is efficacious when it is blended with other fractions.

### CONCLUSION

Recent reports show increased prevalence of antibody positive test results among dogs surveyed for *L. bratislava* exposure. Case studies further support an association between *L. bratislava* and renal and hepatic diseases in dogs in the field. For most leptospiral serovars traditionally associated with renal and hepatic disease, the key to protection has been the prevention of spirochetemia. The present study demonstrates vaccination of *L. bratislava* can be expected to result in similar benefits to canine health.

The results of the current study demonstrate that the outer membrane protein of *L. bratislava,* when blended with the fractions of DA₂PP/CvK/LCIGP (to form DA₂PP/CvK/LCIGPB), is potent and efficacious in six-week old puppies. The minimum antigen load (50 µg/dose) was sufficient to induce protection of vaccinates from spirochetemia due to virulent *L. bratislava* challenge. The efficacy of *L*. *bratislava* in the DA₂PP/CvK/LCIGPB combination product was demonstrated by seroconversion of 100% of vaccinates and none of the controls, and freedom of spirochetemia after virulent challenge in 95% of vaccinates and only 8% of the controls. The reduction in the blood spirochetemia coupled with the seroconversion in the vaccinates as compared to the controls indicates that the other 9 fractions, both killed and modified live antigens, did not interfere with the efficacy of *L*. *bratislava* killed bacterin in DA₂PP/CvK/LCIGPB.

### EXAMPLE 2

### VACCINE COMPRISING LEPTOSPIRA BRATISLAVA, LEPTOSPIRA AUTUMNALIS, LEPTOSPIRA AUSTRALIS, AND LEPTOSPIRA HEBDOMADIS

In this example, a combination vaccine is prepared by first obtaining isolated outer membrane capsular (OMC) polypeptides from each of *L. bratislava, L. autumnalis, L. australis,* and *L*. *hebdomadis* using standard methods available in the art. The OMC polypeptides are then combined in the presence of a veterinary-acceptable diluent at a final concentration of about 50 µg of each OMC polypeptide preparation per 1 ml dose.

40 beagle puppies in an Experimental Group are administered two doses of the combination vaccine by the intraperitoneal route at day 0 and at day 21, while 40 beagle puppies in a Control Group are administered diluent only at the same time points.

Twenty-one days after the second vaccination, the puppies in both the Experimental Group and the Control Group are sub-divided into four Sub-Groups of 10 puppies each. The Sub-Groups are designated A, B, C or D. The puppies in each Sub-Group are challenged with virulent strains of *Leptospira* as shown in the table below:

| | Experimental | | | | Control | | | |
|---|---|---|---|---|---|---|---|---|
| Challenge: | A | B | C | D | A | B | C | D |
| *L. bratislava* | x | | | x | x | | | |
| *L. autumnalis* | | x | | | | x | | |
| *L. australis* | | | x | | | | x | |
| *L. hebdomadis* | | | | x | | | | x |

After challenge, the puppies in both Groups are monitored daily for rectal temperatures and any possible clinical signs associated with infection by either *L*. *bratislava, L. autumnalis, L. australis,* or *L. hebdomadis.* In addition, blood samples are collected daily from each puppy in both Groups for 21 days after challenge. The blood samples are assayed for bacterial cells (*L. bratislava, L. autumnalis, L. australis,* or *L*. *hebdomadis*) and for serum agglutinating antibody to *Leptospira* whole cell antigen using the micro-agglutination assay.

An effective combination vaccine according to this Example will produce one or more of the following effects: (a) the presence of antibodies against at least one of *L*. *bratislava, L. autumnalis, L. australis,* or *L*. *hebdomadis,* with antibody titers of at least between 10 to 500 at 0 to 21 days post-second vaccination (prior to challenge); (b) lower clinical symptoms of *L. bratislava, L. autumnalis, L. australis,* or *L. hebdomadis* infection following challenge with the respective virulent strain(s), as compared to the clinical symptoms observed in control puppies not receiving the combination vaccine; and/or (c) reduced isolation of *L*. *bratislava, L. autumnalis, L. australis,* or *L*. *hebdomadis* live cells from the blood of challenged puppies as compared to the amount of live cells isolated from the blood of control puppies.

### REFERENCES

1. Rentko VT, Clark N, Ross LA, et al. Canine leptospirosis: a retrospective study of 17 cases. J. Vet Intern Med 1992; 6:235-244.

2. Nielsen JN, Cochran GK, Cassels JA, et al. Leptospira interrogans serovar bratislava infection in two dogs. J Am Vet Med Assoc 1991; 3:351-352.

3. Harkin KR, Gartrell CL. Canine leptospirosis in New Jersey and Michigan: 17 cases (1990-1995). J Am Anim Hosp Assoc 1996; 32:495-501.

4. Adin CA and Cowgill LD. Treatment and outcome of dogs with leptospirosis: 36 cases (1990-1998). J Am Vet Med Assoc 2000; 216: 371-375.

All publications and patents mentioned in this specification are indicative of the level of skill of those skilled in the art to which this invention pertains

## Claims

1. An immunogenic composition, comprising an outer membrane capsular (OMC) protein of *Leptospira bratislava,* and further comprising a combination vaccine product containing
(i) a viral immunogen of an attenuated strain *of canine distemper* (CD) virus,
(ii) an attenuated strain of canine adenovirus type 2 (CAV-2),
(iii) an inactivated whole or partial cell preparation of a strain of canine coronavirus (CCV),
(iv) an attenuated strain of canine parainfluenza (CPI) virus, and
(v) an attenuated strain of canine parvovirus (CPV).

2. The composition as claimed in claim 1, further comprising an OMC protein of *Leptospira autumnalis, Leptospira australis,* and *Leptospira hebdomadis.*

3. The composition as claimed in of any one of claims 1 to 2, further comprising an OMC of *Leptospira conicola, Leptospira grippotyphosa,* and *Leptospira icterohaemorrhagiae.*

4. The composition as claimed in any one of claims 2-3, further comprising a *Bordetella* bronchiseptica antigen.

5. The composition as claimed in claim 4, wherein the B. bronchiseptica antigen is a p68 antigen.

6. The composition as claimed in of any one of the preceding claims, wherein the composition is capable of eliciting a protective immune response against *L. bratislava* infection in a host.

7. The composition as claimed in claim 6, wherein the host is a canine, swine, or bovine.

8. The composition as claimed in claim 7, wherein the host is a canine.

9. The composition as claimed in any one of claims 1-8, further comprising a pharmaceutically-or veterinary-acceptable carrier, excipient, or diluent.

10. The composition as claimed in of any one of claims 1 to 9, further comprising a preservative.

11. The composition as claimed in of any one of claims 1 to 10, further comprising an adjuvant.

12. The composition as claimed in claim 11, wherein the preservative is an antibiotic.

13. The composition as claimed in any one of the preceding claims, for use as a vaccine in an animal.

14. The composition as claimed in claim 13 for the use defined in said claim, wherein the animal is a canine

## Patentansprüche

1. Immunogene Zusammensetzung, die ein kapsuläres Außenmembran-Protein (OMC) von *Leptospira bratislava* umfasst und die weiter ein Kombinations-Impfstoff-Produkt umfasst, enthaltend
(i) ein virales Immunogen eines attenuierten Stamms von Hunde-Staupe (CD)-Virus,
(ii) einen attenuierten Stamm von Hunde-Adenovirus-Typ-2 (CAV-2),
(iii) ein inaktivierte Ganz- oder Teil-Zell--Präparat eines Stammes von Hunde-Koronavirus (CCV),
(iv) einen attenuierten Stamm von Hunde-Parainfluenza (CPI)-Virus, und
(v) einen attenuierten Stamm von Hunde-Parvovirus (CPV).

2. Zusammensetzung gemäß Anspruch 1, die weiter ein OMC-Protein von *Leptospira autumnalis, Leptospira australis,* und *Leptospira hebdomadis* umfasst.

3. Zusammensetzung gemäß einem der Ansprüche 1 bis 2, die weiter ein OMC von *Leptospira. canicola, Leptospira grippotyphosa,* und *Leptospira icterohaemorrhagiae* umfasst.

4. Zusammensetzung gemäß einem der Ansprüche 2 bis 3, die weiter ein *Bordetella bronchiseptica*-Antigen umfasst.

5. Zusammensetzung gemäß Anspruch 4, wobei das *B. bronchi*septica-Antigen ein p68-Antigen ist.

6. Zusammensetzung gemäß einem der vorhergehenden Ansprüche, wobei die Zusammensetzung dazu in der Lage ist, eine protektive Immunantwort gegen *L*. *Bratislava*-Infektion in einem Wirt hervorzurufen.

7. Zusammensetzung gemäß Anspruch 6, wobei der Wirt ein Hund, Schwein, oder Rind ist.

8. Zusammensetzung gemäß Anspruch 7, wobei der Wirt ein Hund ist.

9. Zusammensetzung gemäß einem der Ansprüche 1 bis 8, die weiter einen/ein pharmazeutisch oder veterinärmedizinisch verträglichen/verträgliches Träger, Exzipiens, oder Verdünnungsmittel umfasst.

10. Zusammensetzung gemäß einem der Ansprüche 1 bis 9, die weiter ein Konservierungsmittel umfasst.

11. Zusammensetzung gemäß einem der Ansprüche 1 bis 10, die weiter ein Adjuvans umfasst.

12. Zusammensetzung gemäß Anspruch 11, wobei das Konservierungsmittel ein Antibiotikum ist.

13. Zusammensetzung gemäß einem der vorhergehenden Ansprüche zur Verwendung als ein Impfstoff bei einem Tier.

14. Zusammensetzung gemäß Anspruch 13 zur Verwendung, definiert in diesem Anspruch, wobei das Tier ein Hund ist.

## Revendications

1. Composition immunogène comprenant une protéine capsulaire de membrane externe (OMC) de *Leptospira bratislava* et comprenant, en outre, un produit de combinaison vaccinale contenant
(i) un agent immunogène viral d'une souche atténuée de virus de la maladie de Carré (CD),
(ii) une souche atténuée d'adénovirus canin de type 2 (CAV-2),
(iii) une préparation de cellules entières ou partielles inactivées d'une souche de coronavirus canin (CCV),
(iv) une souche atténuée de virus para-influenza canin (CPI), et
(v) une souche atténuée de parvovirus canin (CPV).

2. Composition telle que revendiquée dans la revendication 1, comprenant en outre une protéine OMC de *Leptospira autumnalis, Leptospira australis* et *Leptospira hebdomadis.*

3. Composition telle que revendiquée dans l'une quelconque des revendications 1 à 2, comprenant en outre une OMC de *Leptospira canicola, Leptospira grippotyphosa* et *Leptospira icterohaemorrhagiae.*

4. Composition telle que revendiquée dans l'une quelconque des revendications 2-3, comprenant en outre un antigène de *Bordetella bronchiseptica.*

5. Composition telle que revendiquée dans la revendication 4, dans laquelle l'antigène de *B. bronchiseptica* est un antigène p68.

6. Composition telle que revendiquée dans l'une quelconque des revendications précédentes, dans laquelle la composition est capable de provoquer une réponse immunitaire protectrice contre une infection à *L. bratislava* chez un hôte.

7. Composition telle que revendiquée dans la revendication 6, dans laquelle l'hôte est un canidé, un porc ou un bovin.

8. Composition telle que revendiquée dans la revendication 7, dans laquelle l'hôte est un canidé.

9. Composition telle que revendiquée dans l'une quelconque des revendications 1-8, comprenant en outre un support, excipient ou diluant acceptable au plan pharmaceutique ou vétérinaire.

10. Composition telle que revendiquée dans l'une quelconque des revendications 1 à 9, comprenant en outre un conservateur.

11. Composition telle que revendiquée dans l'une quelconque des revendications 1 à 10, comprenant en outre un adjuvant.

12. Composition telle que revendiquée dans la revendication 11, dans laquelle le conservateur est un antibiotique.

13. Composition telle que revendiquée dans l'une quelconque des revendications précédentes, pour utilisation en tant que vaccin chez un animal.

14. Composition telle que revendiquée dans la revendication 13, en vue de l'utilisation définie dans ladite revendication, dans laquelle l'animal est un canidé.
